# FASCICULE DE BREVET EUROPEEN

(11) **EP 0 403 377 B1**
(45) Date de publication et mention de la délivrance du brevet: **06.12.1995**
(21) Numéro de dépôt: 90401636.7
(22) Date de dépôt: 13.06.1990
(51) Int. Cl.: C08B 37/00, A61K 31/715

(54) **Polysaccharides sulfatés, agent anticoagulant et agent anti-complémentaire obtenus à partir de fucanes d'algues brunes et leur procédé d'obtention**
Sulfatierte Polysaccharide, Antikoagulierungs- und Antikomplementärmittel, hergestellt aus Fukanen aus braunen Algen, und Verfahren zu deren Herstellung
Sulfated polysaccharides, anticoagulant and anticomplementary agent prepared from fucans from brown seaweeds and process for obtaining them

(30) Priorité: 14.06.1989 FR 8907857
(43) Date de publication de la demande: 19.12.1990
(60) Demande divisionnaire: 95105774.4
(73) Titulaire: INSTITUT FRANCAIS DE RECHERCHE POUR L'EXPLOITATION DE LA MER (IFREMER), 75116 Paris (FR)
(72) Inventeur: Colliec, Sylvia, F-75018 Paris (FR); Bretaudiere, Jacqueline, F-75015 Paris (FR); Durand, Patrick, F-44400 Reze (FR); Fischer, Anne-Marie, F-75013 Paris (FR); Jozefonvicz, Jacqueline, F-60260 Lamorlaye (FR); Kloareg, Bernard, F-29250 Saint-Pol-de Leon (FR); Vidal, Catherine, F-75020 Paris (FR)
(74) Mandataire: Ores, Irène

(56) Documents cités:
- GB-A- 890 207
- PATENT ABSTRACTS OF JAPAN, vol. 10, no. 219 (C-363)[2275], 31 juillet 1986;& JP-A-61 057 520
- ACTA CHEMICA SCANDINAVICA, vol. 24, 1970, pages 3339-3352; B. LARSEN et al.: "Sulphated polysaccharides in brown algae"
- CARBOHYDRATE RESEARCH, vol. 186, no. 1, 15 février 1989, pages 119-129,Amsterdam, NL; T. NISHINO et al.: "Isolation, purification, andcharacterization of fucosecontaining sulfated polysaccharides from the brownseaweed Ecklonia kurome and their blood-anticoagulant activities"
- THE JOURNAL OF BIOLOGICAL CHEMISTRY, vol. 264, no. 6, 25 février 1989, pages3618-3623; F.C. CHURCH et al.: "Antithrombin activity of fucoidan"

## Description

La présente invention est relative à de nouveaux polysaccharides sulfatés, obtenus par lyse ménagée des fucanes extraits des Phéophycées (algues brunes), à leur procédé d'obtention, ainsi qu'à un nouvel agent anticoagulant et antithrombotique et un nouvel agent anticomplémentaire.

L'effet anticoagulant est défini comme l'inhibition de la formation de thrombine active dans le plasma, alors que l'effet antithrombotique est défini comme l'inhibition de la formation du *thrombus* et/ou de sa croissance.

Le médicament anticoagulant le plus utilisé actuellement est l'héparine, qui est un polysaccharide sulfaté constitué d'unités glucosamine et acide glucuronique liées en 1-4, dans lesquelles les groupes sulfate sont présents sur la fonction amine de la glucosamine, et/ou sur des fonctions alcool de la glucosamine et de l'acide uronique. L'héparine agit sur la coagulation en potentialisant l'action anticoagulante de deux inhibiteurs plasmatiques. Le premier qui est l'antithrombine III (AT III) agit à la fois sur la thrombine (également connue sous le nom de facteur IIa) et sur le facteur X activé (ou facteur Xa) ; le second, appelé deuxième cofacteur de l'héparine (HC II), agit sur le facteur IIa, et non sur le facteur Xa.

En outre, en ce qui concerne la prévention des thromboses, on utilise de plus en plus des héparines de bas poids moléculaire, obtenues par dépolymérisation ; leur action sur la coagulation globale est faible ; elles n'ont par exemple aucun effet, in vitro, sur le temps de céphaline kaolin (TCK), qui explore de façon globale la coagulation plasmatique par la voie endogène, c'est-à-dire l'ensemble des facteurs plasmatiques à l'exception du facteur VII et des facteurs plaquettaires ; en revanche, elles permettent, in vivo, la prévention des thromboses expérimentales. Il a été montré qu'elles potentialisaient l'activité inhibitrice de l'AT III vis-à-vis du facteur Xa, et ou'elles étaient faiblement actives vis-à-vis du facteur IIa. Toutefois, il semble de plus en plus probable qu'elles exercent essentiellement leur action dans la prévention des thromboses, par leur faible activité anti-IIa résiduelle.

D'autres polysaccharides sont également connus pour leurs propriétés anticoagulantes et/ou antithrombotiques ; il s'agit, par exemple, des dermatanes sulfates, dont l'activité anticoagulante, comparée à celle de l'héparine, est faible, mais dont l'activité anti-thrombotique est équivalente. Ils agissent en inhibant la thrombine, par la potentialisation de l'effet inhibiteur du cofacteur HC II ; en revanche ils n'agissent pas par l'intermédiaire du cofacteur AT III, et sont sans effet sur le facteur Xa.

Le pentosane polysulfate, largement utilisé dans la prévention des thromboses, agit également en catalysant l'inhibition de la thrombine par l'HC II.

Les fucanes sont des polysaccharides sulfatés, de poids moléculaire moyen élevé (100 à 800 kDa), extraits des thalles d'algues brunes. Ce sont des polymères d'α-1,2-L-fucose-4-sulfate pouvant contenir également du D-xylose, du D-galactose et des acides uroniques. Les acides uroniques des fucanes ne sont pas sulfatés, contrairement à ceux de l'héparine. En outre, les fucanes diffèrent à la fois de l'héparine et du dermatane sulfate, en ce qu'ils ne contiennent pas d'amino-sucres.

Des travaux réalisés sur les fucanes bruts, ont permis de séparer, à partir d'un même extrait brut, diverses sous-populations de fucanes, différant les unes des autres par leur poids moléculaire moyen, d'une part, et par leurs propriétés physico-chimiques d'autre part.

En fait, ces travaux, utilisant des procédés non agressifs de fractionnement (précipitation fractionnée, filtration sur gel, etc...) qui ne dégradent pas le squelette polysaccharidique des molécules de fucanes ont mis en évidence l'existence de sous-populations naturelles de fucanes.

LARSEN et al. [Acta. Chem. Scand; 24:9, 3339-3352 (1970)] ont étudié la composition des polysaccharides sulfatés d'*Ascophyllum nodosum* et de *Fucus vésiculosus*. Après hydrolyse par HCl 0,2N à une température de 80°C pendant 60 minutes, du fucane brut extrait de ces algues, trois fractions qui se différencient par leur mobilité en électrophorèse ont été observées.

Ces trois fractions ont été séparées par précipitation fractionnée par l'éthanol en présence de chlorure de magnésium. La fraction soluble dans le chlorure de magnésium (Mgₛ) a été identifiée au fucoïdane ; la fraction insoluble dans le magnésium (Mgᵢ) a été identifiée à l'ascophyllane et la fraction intermédiaire qui est détruite par traitement à l'alginase, a été identifiée à un alginate.

Les propriétés anticoagulantes des fucanes bruts ont été démontrées dès 1957 par SPRINGER et al. et confirmées depuis lors par de nombreux auteurs [BERNARDI et SPRINGER, The Journal of Biological Chemistry, 237.1, (1962) ; MORI et al., Marine Algae in Pharmaceutical Science, 2, 1982].

Une étude réalisée par les inventeurs, sur le mécanisme de l'action du fucane brut sur la coagulation, a montré que le fucane brut allonge le temps de céphaline kaolin (TCK) , et surtout le temps de thrombine (TT) (qui explore la transformation du fibrinogène en caillot de fibrine sous l'influence de la thrombine, dernière phase de la coagulation). Cette étude montre également que le fucane brut agit quasi-exclusivement sur la thrombine (facteur IIa), et ce principalement en potentialisant l'effet de l'inhibiteur HCII (à même concentration que l'héparine), et celui de l'antithrombine III (à des concentrations 30 fois supérieures à celles de l'héparine). En revanche, le fucane n'a, contrairement à l'héparine, aucune activité anti-Xa.

Des observations similaires ont été faites par d'autres auteurs [CHURCH et al. The Journal of Biological Chemistry ; 264.6 (1989)] qui ont également étudié les propriétés du fucane brut. Ces auteurs ont monté que le fucane brut catalyse faiblement l'inhibition de la thrombine par l'AT III, et fortement l'inhibition de la thrombine par l'HC II.

Les fucanes pourraient donc constituer une nouvelle catégorie de médicaments anticoagulants originaux, de par leur mécanisme d'action qui diffère de celui des anticoagulants connus. En effet, ils potentialisent l'activité inhibitrice de l'HC II, et également celle de l'AT III vis-à-vis de la thrombine, contrairement au dermatane sulfate qui potentialise exclusivement l'activité inhibitrice de l'HC II. En revanche, les fucanes, comme le dermatane sulfate et contrairement à l'héparine, n'ont aucun effet sur l'inhibition du facteur Xa ; ils sont des inhibiteurs exclusifs de la thrombine. Les Inventeurs ont également constaté que, de façon surprenante, les fucanes, bien qu'ils ne contiennent (contrairement à l'héparine) ni amino-sucres, ni acides uroniques O-sulfatés, ont la propriété d'inhiber l'activation du complément.

Les inhibiteurs de l'activation du complément peuvent jouer un rôle dans la prévention des phénomènes de rejet des greffes ; leur utilisation a également été suggérée dans le traitement des dialysés rénaux et des convalescents d'infarctus du myocarde. L'activité anticomplémentaire peut également être mise à profit pour inhiber l'activation du complément dans les dispositifs de circulation extra-corporelle

Bien que leurs propriétés anticoagulantes soient connues depuis de longues années, les fucanes bruts n'ont pas été utilisés en thérapeutique, d'une part à cause de leur teneur en protéines résiduelles relativement élevée, qui risque de provoquer des phénomènes immunogènes, et d'autre part, à cause de leur masse moléculaire élevée qui a pour conséquence une mauvaise solubilité, ce qui limite considérablement leur utilisation à de fortes concentrations.

Or, les études réalisées jusqu'à présent sur l'action anticoagulante des fucanes, en particulier celles qui concernaient l'activité anticoagulante des diverses sous-populations de fucane obtenues par fractionnement du fucane brut, suggéraient que l'activité anticoagulante était précisément liée à la grande taille des molécules de fucane. Certaines équipes sont parvenues à la conclusion que l'activité anticoagulante était en relation avec un poids moléculaire élevé. [USUI et al. Agr., Biol. Chem., 44-8, (1980)].

D'autres travaux s'accordent à mettre en évidence l'existence d'une limite inférieure de 20 kDa au-dessous de laquelle aucune activité anticoagulante significative n'a été observée. Le Brevet Anglais 890 207 décrit par exemple l'obtention de 7 fractions différentes, dont les poids moléculaires s'échelonnent entre 5 et 50 000, par précipitation fractionnée à l'éthanol du fucane brut. Selon ce Brevet, parmi ces fractions, seules celles dont le poids moléculaire est supérieur à 20 000 présentent une activité anticoagulante intéressante.

NISHINO et al. [Carbohydrate Research, 186 (1989), 119-129] ont étudié les propriétés anticoagulantes des fractions obtenues à partir de fucanes non dégradés et ont également mis en évidence cette limite de 20 kDa ; en outre, les fractions purifiées décrites dans leur publication ont un mécanisme d'action différent de celui décrit pour des fucanes bruts, car, bien que comme ces derniers, elles semblent être plus actives sur la thrombine que sur le facteur Xa, leur action se manifeste essentiellement au niveau du mécanisme global de la coagulation, ce qui se traduit par un allongement du temps de céphaline kaolin (TCK) ; en revanche, elles n'ont que peu d'effet sur le temps de thrombine, et paraissent donc moins actives au niveau de la dernière phase de la coagulation.

Il apparaît donc que les sous-populations polysaccharidiques obtenues jusqu'alors par fractionnement non-dégradatif du fucane brut ont une action sur la coagulation variable d'une sous-population à une autre, et pouvant être, semble-t-il, différente de celle observée dans le cas du fucane brut ; d'autre part, il ressort des travaux réalisés jusqu'à présent que les fractions de masse moléculaire inférieure à 20 KDa, qui seraient particulièrement avantageuses pour leur solubilité, sont quasiment inactives sur la coagulation.

Les Inventeurs ont émis l'hypothèse qu'en fragmentant les longues chaînes polysaccharidiques du fucane brut, il serait possible d'obtenir des fractions de faible poids moléculaire ; toutefois, la coupure du squelette saccharidique des molécules de fucane nécessite la mise en oeuvre de procédés de lyse qu'il est nécessaire de pouvoir contrôler pour ne pas aboutir à une dégradation trop poussée du fucane, entraînant la perte de ses propriétés.

Les essais déjà effectués en ce sens n'avaient pas permis de mettre au point un procédé de dégradation des fucanes donnant des fractions de poids moléculaire intermédiaire ; en effet, les procédés utilisés aboutissaient soit à une dégradation insuffisante, donnant des fractions de poids moléculaire supérieur à 40 kDa, soit à une dégradation quasi-totale, donnant des fragments dépourvus d'activité anticoagulante. [V.COLLLEC et al., IVèmes Journées d'hémostase et de thrombose, Paris, Mars 1988], [FISCHER et al., International Congress on thrombosis (Athens), Mai 1988], [COLLIEC et al., Polymers in Medicine, Varsovie, Octobre 1988].

La présente invention a en conséquence pour but de pourvoir à des fractions polysaccharidiques de poids moléculaire relativement faible obtenues par lyse ménagée à partir des fucanes d'algues brunes ; elle a également pour but de pourvoir à des agents anticoagulants et antithrombotiques particulièrement intéressants en ce que leur activité s'exerce par inhibition du facteur IIa et en ce qu'ils ne possèdent pas d'activité anti-Xa, et qu'ils ne présentent pas les effets secondaires dus au poids moléculaire élevé et à la haute teneur en protéines résiduelles des fucanes bruts, et oui sont en outre obtenus à partir des algues brunes oui sont des matières premières disponibles aisément en grande quantité. Elle a également pour but de pourvoir à un nouvel agent anticomplémentaire obtenu à partir des fucanes d'algues brunes.

La présente invention a pour objet un procédé d'obtention de polysaccharides sulfatés à partir de fucane brut extrait de Phéophycées, lequel procédé est caractérisé en ce qu'il comprend les étapes suivantes :
- une première étape au cours de laquelle l'on procède à la lyse ménagée dudit fucane brut, par l'action de H₂SO₄ 0,5 à 1N, à une température comprise entre 40° et 50°C, pendant 1 à 4 heures;
- une deuxième étape au cours de laquelle on procède à la filtration sur gel du lysat obtenu, et à l'issue de laquelle l'on recueille des fractions de poids moléculaire supérieur à 5 et inférieur à 40 kDa
La présente Invention a également pour objet des polysaccharides sulfatés qui sont caractérisés en ce qu'ils sont susceptibles d'être obtenus à partir de fucanes de Phéophycées par le procédé conforme a l'Invention, en ce que leur poids moléculaire, déterminé par filtration sur gel par rapport à un étalon polysaccharidique, est supérieur à 5 et inférieur à 40 kDa, en ce que leur teneur en soufre est supérieure à celle du fucane d'origine, en ce qu'ils contiennent moins de 0,15% de protéines contaminantes et en ce qu'ils sont plus solubles que les fucanes d'origine, et en ce qu'ils possédent des propriétés anticoagulantes et antithrombotiques.

L'Invention englobe également d'autres procédés de lyse ménagée de fucanes bruts, qui permettent d'obtenir des fractions de polysaccharides sulfatés conformes à l'Invention, telles que définies ci-dessus. Ces procédés sont les suivants :

Un procédé caractérisé en ce qu'il comprend les étapes suivantes :
- une première étape au cours de laquelle l'on procède à la lyse ménagée dudit fucane brut, par radiolyse ;
- une deuxième étape au cours de laquelle on procède à la filtration sur gel du lysat obtenu, et à l'issue de laquelle l'on recueille des fractions de poids moléculaire supérieur à 5 et inférieur à 40 kDa.

La radiolyse est effectuée en soumettant le fucane à une irradiation, notamment par rayons gamma.
- Un procédé caractérisé en ce qu'il comprend les étapes suivantes :
- une première étape au cours de laquelle l'on procède à la lyse ménagée dudit fucane brut, par hydrolyse enzymatique ;
- une deuxième étape au cours de laquelle on procède à la filtration sur gel du lysat obtenu, et à l'issue de laquelle l'on recueille des fractions de poids moléculaire supérieur à 5 et inférieur à 40 kDa.

Ce procédé implique l'utilisation d'au moins une enzyme capable de fragmenter le squelette hydrocarboné du fucane, par exemple une enzyme du type α-1-2-fucosidase ; des mélanges d'enzymes, qui sont par exemple constitués par des sucs digestifs de mollusques marins ou obtenus à partir de ceux-ci, ou encore par des enzymes contenues dans des bacteries qui dégradent les algues, peuvent également être utilisés.

Selon un mode de mise en oeuvre préféré d'un procédé conforme à l'invention, on sélectionne des fractions dont le poids moléculaire moyen est inférieur ou égal à 20 kDa.

Selon un autre mode de mise en oeuvre préféré d'un procédé conforme à l'invention, on sélectionne des fractions dont le poids moléculaire moyen est compris entre 20 et 35 kDa.

La teneur en soufre des polysaccharides sulfatés obtenus par un procédé conforme à l'invention est supérieure de 2% à 20% à celle des fucanes d'origine.

Des modes de réalisation préférés d'une fraction de polysaccharides sulfatés conformes à l'invention sont les suivants :
- lorsque les polysaccharides sulfatés sont obtenus par hydrolyse acide ménagée à partir des fucanes extraits de *Fucus vesiculosus*, leur teneur en soufre est supérieure d'environ 15% à 20% à celle des fucanes d'origine et leur teneur en protéines est inférieure à 0,05%.
- lorsque les polysaccharides sulfatés sont obtenus par hydrolyse acide ménagée à partir des fucanes extraits d'*Ascophyllum nodosum*, leur teneur en soufre est supérieure d'environ 2% à 5% à celle des fucanes d'origine et leur teneur en protéines est inférieure à 0,O5%.
- lorsque les polysaccharides sulfatés sont obtenus par hydrolyse acide ménagée à partir des fucanes extraits de *Pelvetia canaliculata*, leur teneur en soufre est supérieure d'environ 15% à 20% à celle des fucanes d'origine et leur teneur en protéines est inférieure à 0,05%.
- lorsque les polysaccharides sulfatés sont obtenus par hydrolyse acide ménagée à partir des fucanes extraits de *Undaria pinnatifida*, leur teneur en soufre est supérieure d'environ 1O% à 15% à celle des fucanes d'origine et leur teneur en protéines est inférieure à 0,O5%.

La présente Invention a également pour objet l'utilisation des fractions de polysaccharides sulfatés conformes à l'invention pour l'obtention de médicaments.

Les Inventeurs ont démontré, in vitro et in vivo, que les polysaccharides sulfatés conformes à l'invention possédaient à la fois des propriétés anticoagulantes et antithrombiques. Contrairement aux sous-populations polysaccharidiques de faible poids moléculaire décrites par NISHINO et al., les polysaccharides conformes à l'Invention agissent essentiellement au niveau de la dernière phase de la coagulation.

Des fractions de polysaccharides sulfatés conformes à l'invention peuvent donc être avantageusement utilisées pour l'obtention d'un médicament anticoagulant et antithrombotique, actif aussi bien in vitro qu'in vivo, activateur des co-facteurs HCII et AT III, caractérisé en ce qu'il comprend au moins un polysaccharide sulfaté tel que défini plus haut.

Le poids moléculaire des polysaccharides sulfatés conformes à la présente invention autorise leur solubilisation, même à concentration élevée, dans le plasma et dans les solutions injectables. En outre, ils contiennent, par rapport aux fucanes bruts un faible pourcentage de protéines contaminantes. Il est donc possible d'utiliser ces produits chez l'homme comme médicaments. Ceci est particulièrement avantageux dans certaines applications des propriétés anti-thrombotiques des polysaccharides sulfatés conformes à l'invention, en particulier dans la prévention des thromboses veineuses par injection sous-cutanée d'un antithrombotique ; cette application demande l'utilisation de solutions relativement concentrées, ce qui était impossible avec les fucanes non fractionnés ; il est également envisageable d'utiliser les polysaccharides sulfatés conformes à l'invention par voie orale et transdermique.

La présente invention sera mieux comprise à l'aide du complément de description qui va suivre, et qui se réfère à des exemples d'obtention de polysaccharides sulfatés conformes à la présente invention à partir de fucanes extraits de différentes algues brunes, et à la démonstration des activités de ces polysaccharides.

Il doit être bien entendu, toutefois, que ces exemples sont donnés uniquement à titre d'illustration de l'objet de l'invention, dont ils ne constituent en aucune manière une limitation.

### I - OBTENTION DE FRACTIONS POLYSACCHARIDIQUES SULFATEES CONFORMES A L'INVENTION

### Exemple 1 : Fractionnement des fucanes et purification des fractions obtenues

### A) Extraction de fucanes d'algues brunes.

Les fucanes sont extraits à partir du matériel pariétal des thalles d'algues brunes, par une procédure classique d'extraction organique. Cette procédure est dérivée de la procédure de BLACK et al., [J. Sci. Food Agric. (1952)].

Le protocole d'extraction peut être schématisé comme suit : les thalles sont broyés dans l'éthanol absolu contenant 5% en poids de formol. Le broyat obtenu est soumis à une première extraction, par l'éthanol à 95 % contenant 5% en poids de formol, puis à une deuxième extraction par un mélange acétone/toluène 2/1 (V/V). Après séchage à l'étuve, le matériel obtenu est soumis à une ou plusieurs extractions acides par HCl O,01 M contenant 2% en poids de CaCl₂.

Après neutralisation par la soude, l'extrait acide est traité au chlorure de N-cétylpyridinium qui forme un complexe avec les fucanes les plus sulfatés, et permet donc d'enrichir la préparation en ces derniers. Le précipité obtenu est ensuite redissous dans une solution de CaCl₂3M, puis lyophilisé.

### B) Fractionnement des fucanes extraits.

### 1ère étape : dégradation de l'extrait brut.

### a) par hydrolyse acide

L'extrait acide est dissous à 10 mg/ml dans une solution d'H₂SO₄ 1N et la température fixée à 45°C. La dégradation suivie par viscosimétrie, (viscosimètre de type Ubbelohde de diamètre de capillaire d'écoulement de 0,5 mm) est arrêtée par ajout de soude (NaOH M) avant l'hydrolyse totale du fucane. Au cours de la dégradation, la viscosité spécifique réduite (η spec./c) est calculée toutes les 30 minutes. La solution d'extrait acide dégradé est concentrée, ultrafiltrée en utilisant une membrane ayant un seuil de coupure de 1000 daltons et enfin lyophilisée.

### b) par radiolyse

L'hydrolyse radiochimique est réalisée sous irradiation à partir d'une source de cobalt 60 de 60.000 Ci. Les fucanes bruts sont irradiés à température ambiante pendant 92 heures avec un débit de dose de 2081 rads/minute soit une dose d'irradiation de 11,5 Mrads pour tous les échantillons.

### c) Dégradation enzymatique

La dégradation enzymatique de l'extrait brut de Pelvetia canaliculata par des extraits bruts de suc digestif d'haliotide *(Haliotis tuberculata)* et d'aplysie *(Aplysia californica)* est réalisée comme suit : le fucane brut est dissous à une concentration de 10 mg/mL dans du tampon citrate phosphate ; pH 5,8 (acide citrique = C₆H₈O₇ à 0,1 M et disodium hydrogénophosphate = Na₂HPO₄, 2H₂O à 0,2 M). La solution de fucane est chauffée à 37°C et 200 »l d'extrait digestif sont ajoutés toutes les heures, pour assurer une concentration constante d'enzyme active. La dégradation est suivie par viscosimétrie, la viscosité spécifique réduite, (ηsp)/C, est calculée lors des différents prélèvements. La réaction est arrêtée par addition d'une solution de carbonate disodique (Na₂CO₃)O,2 M.

### 2ème étape : fractionnement préparatif de l'extrait brut dégradé

### a) à l'échelle du laboratoire

500 mg de lyophilisat repris dans 5 ml de NaCl O,2 M sont déposés sur une colonne (hauteur 35 à 45 cm x 4,8 cm) de gel de fractionnement, (SEPHACRYL S-200 ou S-300). Le débit linéaire est de 3,33 cm/heure. Une double détection est réalisée à la sortie de la colonne à l'aide d'un réfractomètre différentiel et d'un détecteur UV à 280 nm. La colonne a été calibrée à l'aide d'étalons polysaccharidiques. Les signaux sont enregistrés sur un enregistreur double voie. Après étude des chromatogrammes, les différentes fractions obtenues à la sortie de la colonne (5 ml) sont réunies en 3 ou 4 fractions. Chaque fraction est concentrée, ultrafiltrée sur une membrane ayant un seuil de coupure de 10 000 daltons pour la première fraction, et une membrane de seuil de coupure de 1 000 daltons pour les autres fractions, et lyophilisée.

### b) à l'échelle semi-pilote

30 g de lyophilisat repris dans 125 ml de NaCl 0,2 M sont déposés sur une colonne de SEPHACRYL S-200 (hauteur 60 cm x diamètre 11,3 cm), le débit linéaire est de 15 cm/heure. Une double détection est réalisée à la sortie de la colonne, à l'aide d'un réfractomètre différentiel et d'un détecteur UV à 280 nm. Les signaux sont enregistrés sur un enregistreur double voie. Après étude des chromatogrammes, les différentes fractions obtenues à la sortie de la colonne (30 ml) sont réunies en 3 ou 4 fractions. Chaque fraction est concentrée, ultrafiltrée sur une membrane ayant un seuil de coupure de 10 000 daltons pour la première fraction, et une membrane de seuil de coupure de 1000 daltons pour les autres fractions, et lyophilisée.

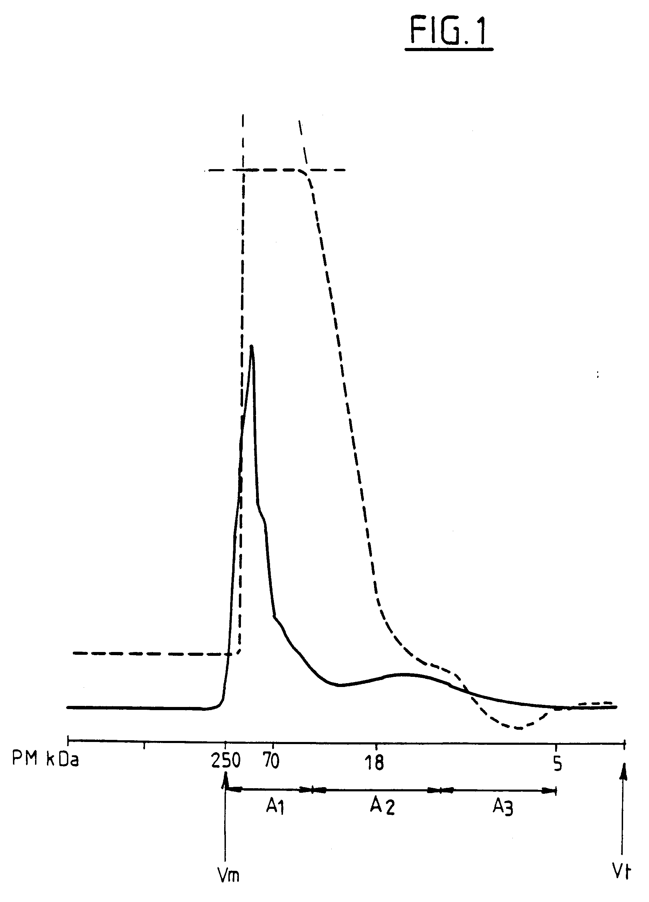

La figure 1 représente le fractionnement préparatif sur gel SEPHACRYL 5-200 (domaine de fractionnement 250 x 10³ à 5 x 10³ daltons) de l'extrait acide de fucane dégradé par H₂SO₄ 1N à 45°C : A₁ = 1ère fraction (PM>20kDa) ; A₂ = 2ème fraction (20 kDa>PM>10kDa) ; A₃ = 3ème fraction (PM<10 kDa) ; Vm = volume mort et Vt = volume total. La courbe en pointillés (----) représente la quantité de matériel élué mesurée par réfractométrie différentielle. La courbe en trait plein (―) représente la quantité de protéines mesurée par absorption UV à 280 nm.

### Exemple 2 : Caractérisation physico-chimique des fractions obtenues

### Spectre infra-rouge :

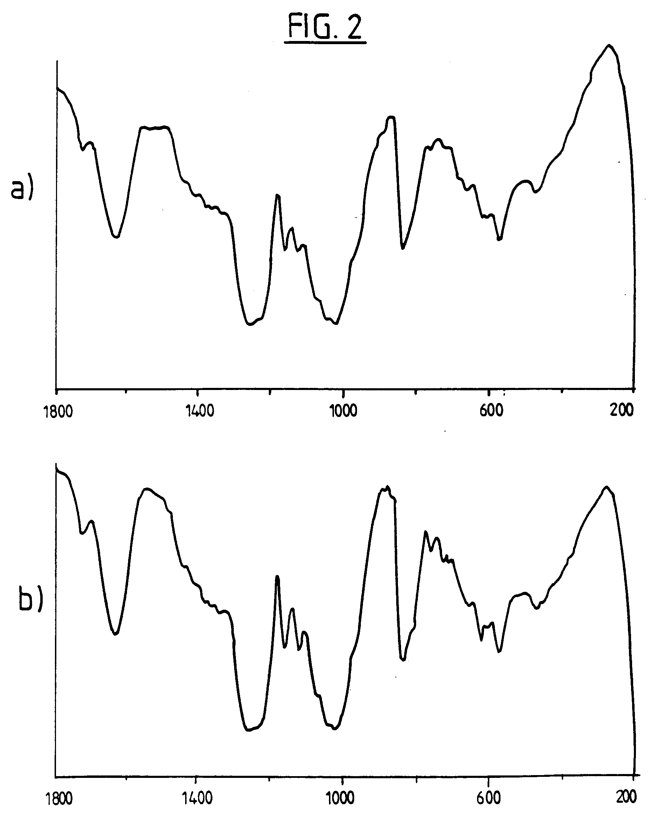

La figure 2 représente les spectres infra-rouge du fucane brut (2a) extrait des parois de *Fucus vesiculosus*, et d'une fraction A₂ (2b) de poids moléculaire moyen 13 x 10³ obtenue par hydrolyse acide ménagée de cet extrait acide. Le pic à 1240 cm⁻¹ est caractéristique des groupes sulfuryle. Le pic à 850 cm⁻¹ montre que la majorité des groupes sulfate est en C₄ sur le L-Fucose ; une partie de ces groupes sulfate est cependant en C₆ comme le montre l'épaulement à 820 cm⁻¹ La majeure partie des fonctions carboxyliques sont sous forme non dissociée comme le montre le pic à 1720 cm⁻¹, comparé au faible épaulement à 1420 cm⁻¹ qui représente les fonctions carboxyliques sous forme dissociée.

### Détermination du poids moléculaire :

La détermination du poids moléculaire en g/mole des fractions de fucane est réalisée en chromatographie liquide haute performance analytique (HPLC) sur une colonne Si-Diol 500 (domaine de fractionnement 5000 à 10⁶ daltons) par injection de 50 »l de polysaccharides étalons (Pullulane des laboratoires POLYMER LABORATORIES LTD.) ou de la fraction de fucane étudiée, à 2 mg/ml dans du NaCl O,2 M, le débit étant de 1 ml/mn et la détection réalisée par réfractométrie. Les chromatogrammes sont stockés puis analysés grâce à un micro-ordinateur possédant un programme GPC (chromatographie d'exclusion stérique sur gel perméable), les résultats sont enregistrés sur une imprimante.

### Teneur en S et N :

La teneur en S et N des fractions de fucane est donnée en % (en poids de produit pur) par analyse élémentaire.

### Teneur en protéines :

La teneur en protéines est estimée en % (en poids de produit pur), par le test colorimétrique du Bradford (test "Bio-Rad") en prenant comme référence l'albumine bovine.

Le tableau I permet d'établir une comparaison entre les propriétés physico-chimiques du fucane brut, c'est-à-dire de l'extrait acide (EA) non fractionné, et des polysaccharides sulfatés conformes à la présente invention, représentés par la fraction A₂ obtenue par hydrolyse acide à partir des fucanes bruts extraits de 4 algues brunes : (*Ascophyllum nodosum* (An), *Fucus vesiculosus* (Fv), *Pelvetia canaliculata* (Pc), *Undaria pinnatifida* (Up).

**Tableau I**

| Poids moléculaire moyen | | S % | protéine % | rendement % |
|---|---|---|---|---|
| EA(An) | 7 x 10⁵ | 8,9 | 0,36 | - |
| A2(An) | 18 x 10³ | 9,1 | <0,01 | 57 |
| EA(Fv) | 8 x 10⁵ | 7,8 | 0,85 | - |
| A2(Fv) | 13 x 10³ | 9,2 | <0,01 | 57 |
| EA(Pc) | 75 x 10⁴ | 9,4 | 0,19 | - |
| A2(Pc) | 18 x 10³ | 11 | <0,01 | 40 |
| EA(Up) | 48 x 10⁴ | 7,2 | 0,26 | - |
| A2(Up) | 23 x 10³ | 8,0 | <0,01 | 36 |

Le tableau II permet d'établir une comparaison entre les propriétés physico-chimiques de l'extrait acide (EA) non fractionné, et des polysaccharides sulfatés conformes à la présente invention, représentés par la fraction A₂ obtenue par radiolyse à partir des fucanes de 3 algues brunes : (*Ascophyllum nodosum* (An), *Fucus vesiculosus* (Fv), *Pelvetia canaliculata* (Pc).

**Tableau II**

| Poids moléculaire Moyen | | S % | protéine % | rendement % |
|---|---|---|---|---|
| EA(An) | 7 x 10⁵ | 8,9 | 0,36 | - |
| A2(An) | 17 x 10³ | 7,8 | 0,11 | 46 |
| EA(Fv) | 8 x 10⁵ | 7,8 | 0,85 | - |
| A2(Fv) | 17 x 10³ | 8,6 | <0,01 | 56 |
| EA(Pc) | 75 x 10⁴ | 9,4 | 0,19 | - |
| A2(Pc) | 15 x 10³ | 9,7 | 0,10 | 58 |
| EA(Up) | 48 x 10⁴ | 7,2 | 0,26 | - |
| A2(Up) | 13 x 10⁴ | 8,0 | 0,11 | 36 |

### II - COMPARAISON DE L'ACTIVITE BIOLOGIQUE DES FUCANES BRUTS ET DES FRACTIONS OBTENUES PAR LYSE MENAGEE DE CEUX-CI

### Exemple 3 : Activité anticoagulante in vitro :

L'activité anticoagulante du fucane non dégradé et de fractions de fucane dégradé de poids moléculaire compris entre 10 et 20 kDa est évaluée par un temps de céphaline-kaolin (TCK). L'activité est donnée en unités internationales par mg de produit (UI/mg), par la relation suivante : (pente de la droite relative de la fraction de fucane/pente de la droite relative de l'héparine étalon) x activité en UI/mg de l'héparine étalon (Héparine H108 des Laboratoires CHOAY).

La pente est calculée à partir de la droite obtenue en reportant le logarithme du temps de coagulation en secondes, en fonction de la concentration en anticoagulant (concentration d'héparine 0,5 à 1 »g/ml de plasma, et de fucane 10 à 50 »g/ml).

Le tableau III permet d'établir une comparaison entre les activités anticoagulantes de l'extrait acide (EA), (ou fucane brut) et de la fraction A₂ (obtenue par hydrolyse acide) des algues brunes : *Ascophyllum nodosum* (An), *Fucus vesiculosus* (Fv), *Pelvetia canaliculata* (Pc) et *Undaria pinnatifida* (Up).

**Tableau III**

| | Activité anticoagulante* (UI/mg) |
|---|---|
| EA(An) | 4,5 |
| A2(An) | 6,6 |
| EA(Fv) | 5,0 |
| A2(Fv) | 5,8 |
| EA(Pc) | 4,0 |
| A2(Pc) | 2,0 |
| EA(Up) | - |
| A2(Up) | 1,3 |

| | |
|---|---|
| * Activité anticoagulante déterminée en prenant pour référence une héparine étalon H108 à 173 UI/mg. | |

Le tableau IV perme d'analyser plus précisément l'action anticoagulante des polysaccharides sulfates conformes à l'invention en permettent la comparaison entre les effets respectifs sur le temps de Quick (TQ), le temps de céphaline kaolin (TCK), le temps de thrombine (TT), et le temps de reptilase (TR), de la fraction A₂ (Pc), et de l'héparine.

**Tableau IV**

| | Temps de coagulation (secondes | | | |
|---|---|---|---|---|
| F2 (PC) (»g/ml) | TQ | TCK | TT | TR |
| 0 | 13 | 56 | 25 | 22 |
| 10 | 14 | 70 | 40 | 22 |
| 25 | 15 | 100 | 130 | 22 |
| 30 | 15 | 120 | >180 | 21 |
| 50 | 15 | 155 | >180 | 22 |
| 100 | 18 | 220 | >180 | 22 |

| Héparine (»g/ml) | TQ | TCK | TT | TR |
|---|---|---|---|---|
| 0,25 | 14 | 85 | 90 | 22 |
| 0,5 | 14 | 120 | >120 | 22 |
| 0,75 | 14 | 150 | >120 | 22 |
| 1 | 14 | >200 | >120 | 22 |
| 5 | 17 | >200 | >120 | 22 |
| 10 | 23 | >200 | >120 | 22 |

Ceci montre que les polysaccharides sulfatés conformes à l'Invention sont essentiellement actifs sur le temps de thrombine.

### Exemple 4 : Activité anticoagulante in vivo ; influence des polysaccharides sulfatés conformes à l'Invention sur les paramètres de la coagulation

L'activité anticoagulante des fractions polysaccharidiques conformes à la présente invention est évaluée sur le lapin. Une fraction de poids moléculaire moyen de 15 000 Da préparée selon le procédé d'hydrolyse acide décrit dans l'exemple 1 est injectée à des lapins de 3 kg. Les lapins sont saignés à intervalles réguliers et les paramètres de la coagulation sont évalués sur le sang prélevé, par la mesure du temps de thrombine (TT) et celle du temps de céphaline kaolin (TCK).

Le tableau V montre l'évolution des paramètres de la coagulation en fonction du temps, après injection de 0,5 ml d'une solution de polysaccharides sulfatés conformes à la présente invention (fraction A2 d'*Ascophyllum nodosum*, obtenue par hydrolyse acide), à 100 mg/ml, 40 mg/ml, 30 mg/ml, et à titre de comparaison après injection d'héparine à 2 mg/ml.

**Tableau V**

| | Temps de prélèvement | TT (sec.) | TCK (sec.) |
|---|---|---|---|
| A₂(An) 100 mg/ml | 0 | 35 | 23,3 |
| | 10mn | >240 | 115 |
| | 30mn | >240 | 88,4 |
| | 4h30 | 31 | 32,4 |
| A₂(An) 40 mg/ml | 0 | 48 | 30 |
| | 10mn | >240 | 73,4 |
| | 30mn | >240 | 58,8 |
| | 4h30 | 23,4 | 25 |
| A₂(An) 30 mg/ml | 0 | 33 | 25 |
| | 10mn | >300 | 59,2 |
| | 30mn | 123,6 | 47 |
| | 4h30 | 20,3 | 24 |
| Héparine | 0 | 28,4 | 23 |
| | 10mn | >240 | 52 |
| | 30mn | >240 | 60 |
| | 4h30 | - | - |

### Exemple 5 : Activité anti-thrombotique in vivo ; thrombose expérimentale

### 1/ Mode opératoire

Le modèle de thrombose expérimentale utilisé est celui de WESSLER et HAUPTMANN [STANFORD WESSLER, STANLEY M. REIMER and MINDEL C. SHEPS - J. Appl. Physiol (1959) 14 p 943-946] chez le lapin, qui utilise le facteur Xa J. HAUPTMANN, B. KAISER, F. MARKWARDT and G. NOUAK - Thromb. Haemostaris Stuttg. 43 (1980) P. 118-123, comme agent déclenchant de la thrombose.

Les essais sont effectués avec une fraction polysaccharidique, de poids moléculaire moyen 20000 ±2000 obtenue par hydrolyse acide du fucane brut, comme décrit dans l'exemple 1. Le produit est injecté par voie intraveineuse aux doses de 0,150 ; 0,625 ; 1,25 ; 2,5 et 5 mg/kg, 10 min avant l'induction de la thrombose. Chaque dose est testée sur 5 lapins. Une détermination de la DE50 (Dose Efficace 50, correspondant à la dose qui diminue de 50% le poids du thrombus formé) antithrombotique a été faite par régression logarithmique, et fait apparaître une valeur moyenne de 0,40 mg/kg (comprise entre 0,23 et 0,66 mg/kg).

Le prélèvement de sang est effectué 10 minutes après l'injection par voie intraveineuse juste après la création du sac veineux. Les résultats moyens obtenus pour chaque dose sont résumés dans le tableau VI.

**Tableau VI**

| Dose injectée (mg/kg) | TCKª (sec) | TT^{b} (sec) | TQ^{c} (sec) |
|---|---|---|---|
| 0 | 30,6 | 20,4 | 8,5 |
| 0,150 | 31,8 | 22,2 | 8,5 |
| 0,625 | 35 | 24,9 | 8,7 |
| 1,25 | 40,2 | 38,5 | 8,6 |
| 2,5 | 50,9 | 45,2 | 8,5 |
| 5 | 67,6 | 71,2 | 8,4 |
| Témoins | 28,8 | 21,4 | 8,3 |

| | | | |
|---|---|---|---|
| a) Temps de céphaline kaolin | | | |
| b) Temps de Thrombine | | | |
| c) Temps de Quick | | | |

Ces résultats confirment ceux observés in vitro et reportés à l'exemple 3 et dans le tableau IV

Les résultats observés sur des lapins témoins soumis au protocole de thrombose expérimentale, mais n'ayant reçu que le solvant de la fraction A₂, sont également indiqués dans ce tableau. Les poids moyens des thrombi humides prélevés dans la veine contrelatérale, pour des doses de 0,15 ; 0,625 ; 1,25 et 5 mg/kg sont indiqués dans le tableau VII.

**Tableau VII**

| | | | | | |
|---|---|---|---|---|---|
| Dose (mg/kg) | 5 | 1,25 | 0,625 | 0,15 | Témoins |
| Poids du Thrombus (mg) | 0,0 | 0,0 | 23,5 | 40 | 76,9 |

## Revendications (Revendications pour l'(les) Etat(s) contractant(s) suivant(s): AT, BE, CH, DE, DK, FR, GB, IT, LI, LU, NL, SE)

1. Procédé d'obtention de polysaccharides sulfatés à partir de fucane brut extrait de Phéophycées, caractérisé en ce qu'il comprend les étapes suivantes :
- une première étape au cours de laquelle l'on procède à la lyse ménagée dudit fucane brut, par l'action de H₂SO₄ 0,5 à 1N, à une température comprise entre 40° et 50°C, pendant 1 à 4 heures;
- une deuxième étape au cours de laquelle on procède à la filtration sur gel du lysat obtenu, et à l'issue de laquelle l'on recueille des fractions de poids moléculaire supérieur à 5 et inférieur à 40 kDa.

2. Procédé selon la revendication 1, caractérisé en ce que le matériel de départ est un fucane brut extrait de *Fucus vesiculosus*.

3. Procédé selon la revendication 1, caractérisé en ce que le matériel de départ est un fucane brut extrait de *Ascophyllum nodosum*.

4. Procédé selon selon la revendication 1, caractérisé en ce que le matériel de départ est un fucane brut extrait de *Pelvetia canaliculata*.

5. Procédé selon selon la revendication 1, caractérisé en ce que le matériel de départ est un fucane brut extrait de *Undaria pinnatifida*.

6. Procédé d'obtention de polysaccharides sulfatés à partir de fucane brut extrait de Phéophycées, caractérisé en ce qu'il comprend les étapes suivantes :
- une première étape au cours de laquelle l'on procède à la lyse ménagée dudit fucane brut, par radiolyse ;
- une deuxième étape au cours de laquelle on procède à la filtration sur gel du lysat obtenu, et à l'issue de laquelle l'on recueille des fractions de poids moléculaire supérieur à 5 et inférieur à 40 kDa.

7. Procédé d'obtention de polysaccharides sulfatés à partir de fucane brut extrait de Phéophycées, caractérisé en ce qu'il comprend les étapes suivantes :
- une première étape au cours de laquelle l'on procède à la lyse ménagée dudit fucane brut, par hydrolyse enzymatique ;
- une deuxième étape au cours de laquelle on procède à la filtration sur gel du lysat obtenu, et à l'issue de laquelle l'on recueille des fractions de poids moléculaire supérieur à 5 et inférieur à 40 kDa.

8. Procédé selon l'une quelconque des revendications 1 à 7, caractérisé en ce que, à l'issue de l'étape de filtration sur gel, l'on recueille les fractions dont le poids moléculaire est compris entre 5 et 20 kDa.

9. Procédé selon l'une quelconque des revendications 1 à 7, caractérisé en ce que, à l'issue de l'étape de filtration sur gel, l'on recueille les fractions dont le poids moléculaire moyen est compris entre 20 et 35 kDa.

10. Fraction de polysaccharides sulfatés obtenue à partir d'un fucane brut extrait de Phéophycées, laquelle fraction est caractérisée en ce qu'elle est susceptible d'être obtenue par un procédé selon une quelconque des revendications 1 à 9, en ce que sa teneur en soufre est supérieure de 2 à 20% à celle du fucane brut d'origine, en ce qu'elle contient moins de 0,15% de protéines.

11. Fraction de polysaccharides sulfatés selon la revendication 10, caractérisée en ce qu'elle est susceptible d'être obtenue par hydrolyse acide ménagée à partir d'un fucane brut extrait de *Fucus vesiculosus*, en ce que sa teneur en soufre est supérieure d'environ 15% à 20% à celle du fucane brut d'origine et en ce que sa teneur en protéines est inférieure à 0,05%.

12. Fraction de polysaccharides sulfatés selon la revendication 10, caractérisée en ce qu'elle est susceptible d'être obtenue par hydrolyse acide ménagée à partir d'un fucane brut extrait de *Ascophyllum nodosum*, en ce que sa teneur en soufre est supérieure d'environ 2% à 5% à celle du fucane brut d'origine et en ce que sa teneur en protéines est inférieure à 0,05%.

13. Fraction de polysaccharides sulfatés selon la revendication 10, caractérisée en ce qu'elle est susceptible d'être obtenue par hydrolyse acide ménagée à partir d'un fucane brut extrait de *Pelvetia canaliculata*, en ce que sa teneur en soufre est supérieure d'environ 15% à 20% à celle du fucane brut d'origine et en ce que sa teneur en protéines est inférieure à 0,05%

14. Fraction de polysaccharides sulfatés selon la revendication 10, caractérisée en ce qu'elle est susceptible d'être obtenue par hydrolyse acide ménagée à partir d'un fucane brut extrait de *Undaria pinnatifida*, en ce que sa teneur en soufre est supérieure d'environ 10% à 15% à celle du fucane brut d'origine et en ce que sa teneur en protéines est inférieure à 0,05%.

15. Utilisation d'une fraction de polysaccharides sulfatés selon l'une quelconque des revendications 10 à 14 pour l'obtention d'un médicament.

16. Utilisation selon la revendication 15, caractérisée en ce que ledit médicament est anticoagulant et antithrombotique, activateur des cofacteurs HCII et ATIII.

## Revendications (Revendications pour l'(les) Etat(s) contractant(s) suivant(s): ES, GR)

1. Procédé d'obtention de polysaccharides sulfatés à partir de fucane brut extrait de Phéophycées, caractérisé en ce qu'il comprend les étapes suivantes :
- une première étape au cours de laquelle l'on procède à la lyse ménagée dudit fucane brut, par l'action de H₂SO₄ 0,5 à 1N, à une température comprise entre 40° et 50°C, pendant 1 à 4 heures;
- une deuxième étape au cours de laquelle on procède à la filtration sur gel du lysat obtenu, et à l'issue de laquelle l'on recueille des fractions de poids moléculaire supérieur à 5 et inférieur à 40 kDa, la teneur en soufre desdites fractions étant supérieure de 2 à 20% à celle du fucane brut d'origine, et leur teneur en protéines étant inférieure à 0,15%.

2. Procédé selon la revendication 1, caractérisé en ce que le matériel de départ est un fucane brut extrait de *Fucus vesiculosus*, en ce que la teneur en soufre de la fraction recueillie à l'issue de l'étape de filtration sur gel est supérieure d'environ 15% à 20% à celle du fucane brut d'origine et en ce que sa teneur en protéines est inférieure à 0,05%.

3. Procédé selon la revendication 1, caractérisé en ce que le matériel de départ est un fucane brut extrait de *Ascophyllum nodosum*, en ce que la teneur en soufre de la fraction recueillie à l'issue de l'étape de filtration sur gel est supérieure d'environ 2% à 5% à celle du fucane brut d'origine et en ce que sa teneur en protéines est inférieure à 0,05%..

4. Procédé selon la revendication 1, caractérisé en ce que le matériel de départ est un fucane brut extrait de *Pelvetia canaliculata*, en ce que la teneur en soufre de la fraction recueillie à l'issue de l'étape de filtration sur gel supérieure d'environ 15% à 20% à celle du fucane brut d'origine et en ce que sa teneur en protéines est inférieure à 0,05%.

5. Procédé selon la revendication 1, caractérisé en ce que le matériel de départ est un fucane brut extrait de *Undaria pinnatifida* en ce que la teneur en soufre de la fraction recueillie à l'issue de l'étape de filtration sur gel supérieure d'environ 10% à 15% à celle du fucane brut d'origine et en ce que sa teneur en protéines est inférieure à 0,05%.

6. Procédé d'obtention de polysaccharides sulfatés à partir de fucane brut extrait de Phéophycées, caractérisé en ce qu'il comprend les étapes suivantes :
- une première étape au cours de laquelle l'on procède à la lyse ménagée dudit fucane brut, par radiolyse ;
- une deuxième étape au cours de laquelle on procède à la filtration sur gel du lysat obtenu, et à l'issue de laquelle l'on recueille des fractions de poids moléculaire supérieur à 5 et inférieur à 40 kDa, la teneur en soufre desdites fractions étant supérieure de 2 à 20% à celle du fucane brut d'origine, et leur teneur en protéines étant inférieure à 0,15%.

7. Procédé d'obtention de polysaccharides sulfatés à partir de fucane brut extrait de Phéophycées, caractérisé en ce qu'il comprend les étapes suivantes :
- une première étape au cours de laquelle l'on procède à la lyse ménagée dudit fucane brut, par hydrolyse enzymatique ;
- une deuxième étape au cours de laquelle on procède à la filtration sur gel du lysat obtenu, et à l'issue de laquelle l'on recueille des fractions de poids moléculaire supérieur à 5 et inférieur à 40 kDa, la teneur en soufre desdites fractions étant supérieure de 2 à 20% à celle du fucane brut d'origine, et leur teneur en protéines étant inférieure à 0,15%.

8. Procédé selon l'une quelconque des revendications 1 à 7, caractérisé en ce que, à l'issue de l'étape de filtration sur gel, l'on recueille les fractions dont le poids moléculaire est compris entre 5 et 20 kDa.

9. Procédé selon l'une quelconque des revendications 1 à 7, caractérisé en ce que, à l'issue de l'étape de filtration sur gel, l'on recueille les fractions dont le poids moléculaire moyen est compris entre 20 et 35 kDa.

10. Procédé de préparation d'un médicament, caractérisé en ce que l'on utilise pour cette préparation une fraction de polysaccharides sulfatés selon l'une quelconque des revendications 1 à 9.

11. Procédé selon la revendication 10, caractérisé en ce que ledit médicament est anticoagulant et antithrombotique, activateur des cofacteurs HCII et ATIII.

## Claims (Claims for the following Contracting State(s): AT, BE, CH, DE, DK, FR, GB, IT, LI, LU, NL, SE)

1. Process for obtaining sulphated polysaccharides from crude fucan extracted from Phaeophyceae, characterized in that it comprises the following stages:
- a first stage in which the said crude fucan is subjected to controlled lysis, by the action of 0.5 to 1 N H₂SO₄, at a temperature of between 40° and 50°C, for 1 to 4 hours;
- a second stage in which the lysate obtained is subjected to gel filtration, and at the end of which fractions with a molecular weight greater than 5 and less than 40 kDa are recovered.

2. Process according to Claim 1, characterized in that the starting material is a crude fucan extracted from *Fucus vesiculosus*.

3. Process according to Claim 1, characterized in that the starting material is a crude fucan extracted from *Ascophyllum nodosum*.

4. Process according to Claim 1, characterized in that the starting material is a crude fucan extracted from *Pelvetia canaliculata*.

5. Process according to Claim 1, characterized in that the starting material is a crude fucan extracted from *Undaria pinnatifida*.

6. Process for obtaining sulphated polysaccharides from crude fucan extracted from Phaeophyceae, characterized in that it comprises the following stages:
- a first stage in which the said crude fucan is subjected to controlled lysis, using radiolysis:
- a second stage in which the lysate obtained is subjected to gel filtration, and at the end of which fractions with a molecular weight greater than 5 and less than 40 kDa are recovered.

7. Process for obtaining sulphated polysaccharides from crude fucan extracted from Phaeophyceae, characterized in that it comprises the following stages:
- a first stage in which the said crude fucan is subjected to lysis, using enzymatic hydrolysis:
- a second stage in which the lysate obtained is subjected to gel filtration, and at the end of which fractions with a molecular weight greater than 5 and less than 40 kDa are recovered.

8. Process according to any one of Claims 1 to 7, characterized in that, at the end of the gel filtration stage, the fractions whose molecular weight is between 5 and 20 kDa are recovered.

9. Process according to any one of Claims 1 to 7, characterized in that, at the end of the gel filtration stage, the fractions whose mean molecular weight is between 20 and 35 kDa are recovered.

10. Sulphated polysaccharide fraction obtained from a crude fucan extracted from Phaeophyceae, which fraction is characterized in that it can be obtained by a process according to any one of Claims 1 to 9, in that its sulphur content is 2 to 20% greater than that of the original crude fucan, in that it contains less than 0.15% protein.

11. Sulphated polysaccharide fraction according to Claim 10, characterized in that it can be obtained by controlled acid hydrolysis from a crude fucan extracted from *Fucus vesiculosus*, in that its sulphur content is about 15% to 20% greater than that of the original crude fucan and in that its protein content is less than 0.05%.

12. Sulphated polysaccharide fraction according to Claim 10, characterized in that it can be obtained by controlled acid hydrolysis from a crude fucan extracted from *Ascophyllum nodosum*, in that its sulphur content is about 2% to 5% greater than that of the original crude fucan and in that its protein content is less than 0.05%.

13. Sulphated polysaccharide fraction according to Claim 10, characterized in that it can be obtained by controlled acid hydrolysis from a crude fucan extracted from *Pelvetia canaliculata*, in that its sulphur content is about 15% to 20% greater than that of the original crude fucan and in that its protein content is less than 0.05%.

14. Sulphated polysaccharide fraction according to Claim 10, characterized in that it can be obtained by controlled acid hydrolysis from a crude fucan extracted from *Undaria pinnatifida*, in that its sulphur content is about 10% to 15% greater than that of the original crude fucan and in that its protein content is less than 0.05%.

15. Use of a sulphated polysaccharide fraction according to any one of Claims 10 to 14 for obtaining a medicinal product.

16. Use according to Claim 15, characterized in that the said medicinal product is an anticoagulant and an antithrombotic, an activator of cofactors HCII and ATIII.

## Claims (Claims for the following Contracting State(s): ES, GR)

1. Process for obtaining sulphated polysaccharides from crude fucan extracted from Phaeophyceae, characterized in that it comprises the following stages:
- a first stage in which the said crude fucan is subjected to controlled lysis, by the action of 0.5 to 1 N H₂SO₄, at a temperature of between 40° and 50°C, for 1 to 4 hours;
- a second stage in which the lysate obtained is subjected to gel filtration, and at the end of which fractions with a molecular weight greater than 5 and less than 40 kDa are recovered, the sulphur content of the said fractions being 2 to 20% greater than that of the original crude fucan, and their protein content being less than 0.15%.

2. Process according to Claim 1, characterized in that the starting material is a crude fucan extracted from *Fucus vesiculosus*, in that the sulphur content of the fraction recovered at the end of the gel filtration stage is about 15% to 20% greater than that of the original crude fucan and in that its protein content is less than 0.05%.

3. Process according to Claim 1, characterized in that the starting material is a crude fucan extracted from *Ascophyllum nodosum*, in that the sulphur content of the fraction recovered at the end of the gel filtration stage is about 2% to 5% greater than that of the original crude fucan and in that its protein content is less than 0.05%.

4. Process according to Claim 1, characterized in that the starting material is a crude fucan extracted from *Pelvetia canaliculata*, in that the sulphur content of the fraction recovered at the end of the gel filtration stage is about 15% to 20% greater than that of the original crude fucan and in that its protein content is less than 0.05%.

5. Process according to Claim 1, characterized in that the starting material is a crude fucan extracted from *Undaria pinnatifida*, in that the sulphur content of the fraction recovered at the end of the gel filtration stage is about 10% to 15% greater than that of the original crude fucan and in that its protein content is less than 0.05%.

6. Process for obtaining sulphated polysaccharides from crude fucan extracted from Phaeophyceae, characterized in that it comprises the following stages:
- a first stage in which the said crude fucan is subjected to controlled lysis, using radiolysis;
- a second stage in which the lysate obtained is subjected to gel filtration, and at the end of which fractions with a molecular weight greater than 5 and less than 40 kDa are recovered, the sulphur content of the said fractions being 2 to 20% greater than that of the original crude fucan, and their protein content being less than 0.15%.

7. Process for obtaining sulphated polysaccharides from crude fucan extracted from Phaeophyceae, characterized in that it comprises the following stages:
- a first stage in which the said crude fucan is subjected to controlled lysis, using enzymatic hydrolysis:
- a second stage in which the lysate obtained is subjected to gel filtration, and at the end of which fractions with a molecular weight greater than 5 and less than 40 kDa are recovered, the sulphur content of the said fractions being 2 to 20% greater than that of the original crude fucan, and their protein content being less than 0.15%.

8. Process according to any one of Claims 1 to 7, characterized in that, at the end of the gel filtration stage, the fractions whose molecular weight is between 5 and 20 kDa are recovered.

9. Process according to any one of Claims 1 to 7, characterized in that, at the end of the gel filtration stage, the fractions whose mean molecular weight is between 20 and 35 kDa are recovered.

10. Process for preparing a medicinal product, characterized in that a sulphated polysaccharide fraction according to any one of Claims 1 to 9 is used for this preparation.

11. Process according to Claim 10, characterized in that the said medicinal product is an anticoagulant and an antithrombotic, an activator of cofactors HCII and ATIII.

## Patentansprüche (Patentansprüche für folgende(n) Vertragsstaat(en): AT, BE, CH, DE, DK, FR, GB, IT, LI, LU, NL, SE)

1. Verfahren zur Isolierung von sulfatierten Polysacchariden aus rohem Fukan, extrahiert aus Pheophyceen, **gekennzeichnet** durch die folgenden Stufen:
- eine erste Stufe, im Verlauf derer man das Rohfukan durch 0,5 bis 1 N H₂SO₄ bei einer Temperatur zwischen 40° und 50°C während 1 bis 4 Stunden schonend lysiert;
- eine zweite Stufe, im Verlauf derer man das so erhaltene Lysat gelfiltriert und nach Abschluß derer man Fraktionen mit einem Molekulargewicht über 5 und unter 40 kDa isoliert.

2. Verfahren nach Anspruch 1, dadurch **gekennzeichnet,** daß das Ausgangsmaterial ein Rohfukan, extrahiert aus Fucus vesiculosus, ist.

3. Verfahren nach Anspruch 1, dadurch **gekennzeichnet,** daß das Ausgangsmaterial ein Rohfukan, extrahiert aus Ascophyllum nodosum, ist.

4. Verfahren nach Anspruch 1, dadurch **gekennzeichnet,** daß das Ausgangsmaterial ein Rohfukan, extrahiert aus Pelvetia canaliculata, ist.

5. Verfahren nach Anspruch 1, dadurch **gekennzeichnet,** daß das Ausgangsmaterial ein Rohfukan, extrahiert aus Undaria pinnatifida, ist.

6. Verfahren zur Isolierung von sulfatierten Polysacchariden aus Rohfukan, extrahiert aus Pheophyceen, **gekennzeichnet** durch die folgenden Stufen:
- eine erste Stufe, im Verlauf derer man das Rohfukan durch Radiolyse schonend lysiert;
- eine zweite Stufe, im Verlauf derer man das so erhaltene Lysat gelfiltriert und am Ende derer man Fraktionen mit einem Molekulargewicht über 5 und kleiner als 40 kDa isoliert.

7. Verfahren zur Isolierung von sulfatierten Polysacchariden aus Rohfukan, extrahiert aus Pheophyceen, **gekennzeichnet** durch die folgenden Stufen:
- eine erste Stufe, im Verlauf derer man das Rohfukan durch enzymatische Hydrolyse schonend lysiert;
- eine zweite Stufe, im Verlauf derer man das so erhaltene Lysat gelfiltriert und am Ende derer man Fraktionen mit einem Molekulargewicht über 5 und kleiner als 40 kDa isoliert.

8. Verfahren nach einem der Ansprüche 1 bis 7, dadurch **gekennzeichnet,** daß man am Ende der Gelfiltrationsstufe die Fraktionen isoliert, deren Molekulargewicht zwischen 5 und 20 kDa liegt.

9. Verfahren nach einem der Ansprüche 1 bis 7, dadurch **gekennzeichnet,** daß man am Ende der Gelfiltrationsstufe die Fraktionen isoliert, deren mittleres Molekulargewicht zwischen 20 und 35 kDa liegt.

10. Fraktion von sulfatierten Polysacchariden, erhalten aus einem Rohfukan, extrahiert aus Pheophyceen, dadurch **gekennzeichnet,** daß sie durch ein Verfahren nach einem der Ansprüche 1 bis 9 erhalten werden kann, daß ihr Schwefelgehalt über 2 bis 20%, bezogen auf den des rohen Ausgangsfukans, liegt, daß sie weniger als 0,15% Proteine enthält.

11. Fraktion von sulfatierten Polysacchariden nach Anspruch 10, dadurch **gekennzeichnet,** daß sie durch schonende saure Hydrolyse aus einem Rohfukan, extrahiert aus Fucus vesiculosus, erhalten werden kann, daß ihr Schwefelgehalt über etwa 15% bis 20%, bezogen auf den des rohen Ausgangsfukans, liegt, und daß ihr Proteingehalt unter 0,05% liegt.

12. Fraktion von sulfatierten Polysacchariden nach Anspruch 10, dadurch **gekennzeichnet,** daß sie durch schonende saure Hydrolyse aus einem Rohfukan, extrahiert aus Ascophyllum nodosum, erhalten werden kann, daß ihr Schwefeigehalt über etwa 2% bis 5%, bezogen auf den des rohen Ausgangsfukans, liegt, und daß ihr Proteingehalt unter 0,05% liegt.

13. Fraktion von sulfatierten Polysacchariden nach Anspruch 10, dadurch **gekennzeichnet,** daß sie durch schonende saure Hydrolyse aus einem Rohfukan, extrahiert aus Pelvetia canaliculata, erhalten werden kann, daß ihr Schwefelgehalt über etwa 15% bis 20%, bezogen auf den des rohen Ausgangsfukans, liegt, und daß ihr Proteingehalt unter 0,05% liegt.

14. Fraktion von sulfatierten Polysacchariden nach Anspruch 10, dadurch **gekennzeichnet,** daß sie durch schonende saure Hydrolyse aus einem Rohfukan, extrahiert aus Undaria pinnatifida, erhalten werden kann, daß ihr Schwefelgehalt über etwa 10% bis 15%, bezogen auf den des rohen Ausgangsfukans, liegt, und daß ihr Proteingehalt unter 0,05% liegt.

15. Verwendung einer Fraktion von sulfatierten Polysacchariden nach einem der Ansprüche 10 bis 14 zum Erhalt eines Medikaments.

16. Verwendung nach Anspruch 15, dadurch **gekennzeichnet,** daß das Medikament ein Antikoagulans und Antithromboticum, ein Aktivator der Co-Faktoren HCII und ATIII ist.

## Patentansprüche (Patentansprüche für folgende(n) Vertragsstaat(en): ES, GR)

1. Verfahren zur Isolierung von sulfatierten Polysacchariden aus rohem Fukan, extrahiert aus Pheophyceen, **gekennzeichnet** durch die folgenden Stufen:
- eine erste Stufe, im Verlauf derer man das Rohfukan durch 0,5 bis 1 N H₂SO₄ bei einer Temperatur zwischen 40° und 50°C während 1 bis 4 Stunden schonend lysiert;
- eine zweite Stufe, im Verlauf derer man das so erhaltene Lysat gelfiltriert und nach Abschluß derer man Fraktionen mit einem Molekulargewicht über 5 und unter 40 kDa isoliert, wobei der Schwefelgehalt der Fraktionen über 2 bis 20%, bezogen auf den des rohen Ausgangsfukans, liegt und ihr Proteingehalt unter 0,15% liegt.

2. Verfahren nach Anspruch 1, dadurch **gekennzeichnet,** daß das Ausgangsmaterial ein Rohfukan, extrahiert aus Fucus vesiculosus, ist, daß der Schwefelgehalt der am Ende der Gelfiltrationsstufe erhaltenen Fraktion über etwa 15% bis 20%, bezogen auf den des rohen Ausgangsfukans, liegt, und daß sein Proteingehalt unter 0,05% liegt.

3. Verfahren nach Anspruch 1, dadurch **gekennzeichnet,** daß das Ausgangsmaterial ein Rohfukan, extrahiert aus Ascophyllum nodosum, ist, daß der Schwefelgehalt der am Ende der Gelfiltrationsstufe erhaltenen Fraktion über etwa 2% bis 5%, bezogen auf den des rohen Ausgangsfukans, liegt, und daß sein Proteingehalt unter 0,05% liegt.

4. Verfahren nach Anspruch 1, dadurch **gekennzeichnet,** daß das Ausgangsmaterial ein Rohfukan, extrahiert aus Pelvetia canaliculata, ist, daß der Schwefelgehalt der am Ende der Gelfiltrationsstufe erhaltenen Fraktion über etwa 15% bis 20%, bezogen auf den des rohen Ausgangsfukans, liegt, und daß sein Proteingehalt unter 0,05% liegt.

5. Verfahren nach Anspruch 1, dadurch **gekennzeichnet,** daß das Ausgangsmaterial ein Rohfukan, extrahiert aus Undaria pinnatifida, ist, daß der Schwefelgehalt der am Ende der Gelfiltrationsstufe erhaltenen Fraktion über etwa 10% bis 15%, bezogen auf den des rohen Ausgangsfukans, liegt, und daß sein Proteingehalt unter 0,05% liegt.

6. Verfahren zur Isolierung von sulfatierten Polysacchariden aus Rohfukan, extrahiert aus Pheophyceen, **gekennzeichnet** durch die folgenden Stufen:
- eine erste Stufe, im Verlauf derer man das Rohfukan durch Radiolyse schonend lysiert;
- eine zweite Stufe, im Verlauf derer man das so erhaltene Lysat gelfiltriert und am Ende derer man Fraktionen mit einem Molekulargewicht über 5 und kleiner als 40 kDa isoliert, wobei der Schwefelgehalt der Fraktionen über 2 bis 20%, bezogen auf den des rohen Ausgangsfukans, liegt, und ihr Proteingehalt unter 0,15% liegt.

7. Verfahren zur Isolierung von sulfatierten Polysacchariden aus Rohfukan, extrahiert aus Pheophyceen, **gekennzeichnet** durch die folgenden Stufen:
- eine erste Stufe, im Verlauf derer man das Rohfukan durch enzymatische Hydrolyse schonend lysiert;
- eine zweite Stufe, im Verlauf derer man das so erhaltene Lysat gelfiltriert und am Ende derer man Fraktionen mit einem Molekulargewicht über 5 und kleiner als 40 kDa isoliert, wobei der Schwefelgehalt der Fraktionen über 2 bis 20%, bezogen auf den des rohen Ausgangsfukans, liegt, und ihr Proteingehalt unter 0,15% liegt.

8. Verfahren nach einem der Ansprüche 1 bis 7, dadurch **gekennzeichnet,** daß man am Ende der Gelfiltrationsstufe die Fraktionen isoliert, deren Molekulargewicht zwischen 5 und 20 kDa liegt.

9. Verfahren nach einem der Ansprüche 1 bis 7, dadurch **gekennzeichnet,** daß man am Ende der Gelfiltrationsstufe die Fraktionen isoliert, deren mittleres Molekulargewicht zwischen 20 und 35 kDa liegt.

10. Verfahren zur Herstellung eines Medikaments, dadurch **gekennzeichnet,** daß man für diese Herstellung eine Fraktion von sulfatierten Polysacchariden nach einem der Ansprüche 1 bis 9 verwendet.

11. Verfahren nach Anspruch 10, dadurch **gekennzeichnet,** daß das Medikament ein Antikoagulans und Antithromboticum, ein Aktivator der Co-Faktoren HCII und ATIII ist.
